# EUROPÄISCHE PATENTSCHRIFT

(11) **EP 2 588 020 B1**
(45) Veröffentlichungstag und Bekanntmachung des Hinweises auf die Patenterteilung: **21.02.2018**
(21) Anmeldenummer: 11743945.5
(22) Anmeldetag: 29.06.2011
(51) Int. Cl.: A61C 5/00, A61K 6/02

(54) **ZAHNFRONTVERBLENDUNGSKÖRPER**
TOOTH FRONT VENEER
CORPS FACETTE DENTAIRE

(30) Priorität: 02.07.2010 AT 11242010
(43) Veröffentlichungstag der Anmeldung: 08.05.2013
(60) Teilanmeldung: 15000991.8
(73) Patentinhaber: Lampl, Stephan, 9451 Kriessern (CH)
(72) Erfinder: Lampl, Stephan, 9451 Kriessern (CH)
(74) Vertreter: Fechner, Thomas
(86) Internationale Anmeldenummer: PCT/AT2011/000285
(87) Internationale Veröffentlichungsnummer: WO 2012/000006

(56) Entgegenhaltungen:
- DE-A1- 10 234 994
- US-A1- 2004 096 805

## Beschreibung

Die vorliegende Erfindung betrifft einen Zahnfrontverblendungskörper, bestehend aus einem zusammengesetzten Material, wobei das zusammengesetzte Material zumindest ein organisches Bindemittel und anorganische Feststoffpartikel als Füllstoff enthält.

Zahnfrontverblendungskörper sind beim Stand der Technik bekannt. Sie werden häufig auch als Veneer bezeichnet. Es handelt sich um meist schalenförmig ausgebildete Festkörper, welche als Verblendung auf gegebenenfalls vorher abgeschliffene Zähne bzw. Zahnstümpfe aufgesetzt werden, um dem verfärbten Zahn wieder die gewünschte Optik zu geben. Sie ersetzen in der Regel somit die natürliche Frontfläche des jeweiligen Zahns.

Beim Stand der Technik werden diese Zahnfrontverblendungskörper aus Keramik gefertigt. Hierzu wird zunächst am abgeschliffenen Zahn bzw. Zahnstumpf z.B. mittels Abdruck die benötigte Geometrie des Zahnfrontverblendungskörpers bestimmt. Nach diesen Angaben wird dann der keramische Zahnfrontverblendungskörper im Labor gefertigt. In einem dritten Schritt muss dann der Zahnarzt diesen Zahnfrontverblendungskörper am Zahn bzw. Zahnstumpf anbringen. Wurde der keramische Zahnfrontverblendungskörper nicht mit der notwendigen Sorgfalt gefertigt, so muss ein neuer Zahnfrontverblendungskörper angefertigt werden, da eine Anpassung an die Gegebenheiten im Mund des Patienten vor Ort beim Zahnarzt nicht möglich ist. Dies bedeutet in Summe, dass der Patient zumindest zweimal zum Zahnarzt gehen muss, bis der passende Zahnfrontverblendungskörper hergestellt und am Zahn angebracht ist. Weiters bedeutet die Notwendigkeit der Laborarbeit einen erheblichen Zeit- und Kostenaufwand.

Die US 2004/0096805 A1 offenbart einen Zahnfrontverblendungskörper, welcher aus einem zusammengesetzten Material aus organischem Bindemittel und anorganischen Feststoffpartikeln als Füllstoff besteht und auf einen bearbeiteten Zahnstumpf aufgebracht wird. Die gattungsgemäße DE 102 34 994 A1 schlägt vorgefertigte aber plastisch noch verformbare Zahnverblendungen vor, welche nach dem Aufbringen auf den abgeschliffenen Zahn noch in ihrer Form angepasst und durch Bestrahlung mit Licht im Mundraum des Patienten polymerisiert und ausgehärtet werden.

Aufgabe der Erfindung ist es, einen alternativen Zahnfrontverblendungskörper zur Verfügung zu stellen, welcher bei einem einzigen Zahnarztbesuch in seiner Größe angepasst und am schadhaften Zahn bzw. Zahnstumpf angebracht werden kann. Erfindungsgemäß wird vorgeschlagen, dass eine äußere Oberfläche des Zahnfrontverblendungskörpers zumindest bereichsweise laserbehandelt ist und eine durchgehende ausgehärtete bzw. verglaste Schmelzschicht bildet, welche im Wesentlichen nur aus dem verschmolzenen Material der Feststoffpartikel besteht. Zahnfrontverblendungskörper dieser Art können in ihrer Form vor Ort vom Zahnarzt mit den in seiner Praxis üblichen Werkzeugen bearbeitet und damit an die beim Patient vorhandenen Vorgaben angepasst werden. Es ist somit nicht mehr notwendig, die Zahnfrontverblendungskörper im Labor herstellen zu lassen. Wie beim Stand der Technik handelt es sich bei den erfindungsgemäßen Zahnfrontverblendungskörpern bzw. Veneers um Festkörper, welche auf einen vorab wie beim Stand der Technik bekannt bearbeiteten Zahnstumpf bzw. Zahn aufgesetzt werden können, um die Frontfläche eines verfärbten Zahnes entsprechend zu verblenden und optisch aufzuwerten. Vor allem auf Grund der als Füllstoff verwendeten Feststoffpartikel weist der Zahnfrontverblendungskörper aber auch die notwendige Festigkeit bzw. Härte auf, damit der mit dem Zahnfrontverblendungskörper versehene Zahn dauerhaft seiner eigentlichen Aufgabe als Kauwerkzeug nachkommen kann.

Günstig ist es in diesem Zusammenhang insbesondere, wenn die Feststoffpartikel des Füllstoffs Glas, vorzugsweise eine Mischung verschiedener Glasarten, aufweisen oder daraus bestehen. Die Glasarten können sich sowohl in ihrer Färbung als auch in ihrer Zusammensetzung unterscheiden. Z.B. ist es denkbar Bariumgläser oder Strontiumgläser oder Gemische daraus zu verwenden. Allgemein kann es sich um oberflächenbehandeltes SiO2 handeln. Um eine hohe Festigkeit des Zahnfrontverblendungskörpers zu erreichen, ist es günstig, die Feststoffpartikel des Füllstoffes in einer möglichst dichten Packung im Zahnfrontverblendungskörper anzuordnen. Hierzu ist es günstig, wenn der Füllstoff Feststoffpartikel mit voneinander verschiedenen Korngrößen aufweist. Generell gesprochen sehen bevorzugte Ausgestaltungsformen vor, dass die Korngrößen der Feststoffpartikel des Füllstoffs in einem Bereich zwischen 0,01 µm und 50 µm liegen. Um eine möglichst hohe Packungsdichte zu erreichen, sind aber insbesondere auch die kleinen Korngrößen wichtig, da sie die Zwischenräume zwischen den größeren Körnern füllen. In diesem Sinne ist es günstig, wenn der Füllstoff Feststoffpartikel mit Korngrößen zwischen 0,01 µm und 3 µm aufweist. Im Sinne der Festigkeit ist weiters ein möglichst hoher Füllgrad des zusammengesetzten Materials des Zahnfrontverblendungskörpers anzustreben. Der Anteil der Feststoffpartikel des Füllstoffs am zusammengesetzten Material sollte also möglichst hoch sein. Günstige Varianten der Erfindung sehen in diesem Zusammenhang vor, dass die Feststoffpartikel des Füllstoffs einen Anteil von zumindest 75 Vol-%, vorzugsweise von zumindest 82 Vol-%, am zusammengesetzten Material des Zahnfrontverblendungskörpers haben.

Als organische Bindemittel verwenden bevorzugte Ausgestaltungsformen der Erfindung Methacrylat basierte Bindemittel. Diese können aus Methacrylat bestehen oder Methacrylat, vorzugsweise als Hauptbestandteil, aufweisen.

Bei den Zahnfrontverblendungskörpern handelt es sich günstigerweise um Festkörper, welche bevorzugt zumindest bereichsweise schalenförmig gewölbt ausgebildet sind.

Eine wichtige Aufgabe beim Anbringen von Zahnfrontverblendungskörpern an Zähnen bzw. Zahnstümpfen ist es, die Farbgebung des mit dem Zahnfrontverblendungskörpers verblendeten Zahns so auszubilden, dass sie an die Gegebenheiten im Mund des Patienten, also auch an die benachbarten Zähne angepasst ist. Bevorzugte Ausgestaltungsformen der Erfindung sehen vor, dass der Zahnfrontverblendungskörper standardisiert eine Farbe aufweist, welche der Farbe von natürlichem Zahnschmelz nahekommt. Besonders bevorzugt ist in diesem Sinne vorgesehen, dass der Zahnfrontverblendungskörper transluzent bzw. durchscheinend, also nicht opak ausgebildet ist. Eine Idee besteht darin, die Farbanpassung vorzunehmen, indem man ein jeweils entsprechend eingefärbtes Befestigungsmaterial verwendet, welches beim Anbringen des Zahnfrontverblendungskörpers zwischen diesem und dem Zahn bzw. Zahnstumpf angeordnet ist und durch den transluzenten Zahnfrontverblendungskörper hindurchscheint, und so die endgültige, nach außen hin wahrnehmbare Farbe des Gesamtaufbaus prägt. Untersuchungen haben ergeben, dass der Zahnfrontverblendungskörper günstigerweise eine Transluzenz von 28% bis 39% und/oder einen L-Wert von 59 bis 69 und/oder einen a-Wert von -0,35 bis -3,2 und/oder einen b-Wert von -0,4 bis -6,95 aufweist. Bei der Transluzenz handelt es sich um die Lichtdurchlässigkeit, also die reziproke Eigenschaft zur Opazität. Die genannten Werte beziehen sich auf eine Messung gemäß DIN 6174. Auch der Lab-Farbraum ist beim Stand der Technik bekannt. Er ist auf Grundlage der Gegenfarbentheorie konstruiert und ermöglicht die farbmetrische Bestimmung von Farbmaßzahlen und Farbabständen im angenähert gleichförmigen CIELAB-Farbenraum. Die Bestimmung des L-Werts, des a-Werts und des b-Werts erfolgt ebenfalls gemäß den Vorgaben von DIN 6174. In Anwendung der Gegenfarbentheorie liegen sich hier Grün und Rot auf der a-Achse gegenüber. Die b-Achse entspricht den Gegenfarben Blau und Gelb. Die L-Achse steht auf dieser Ebene senkrecht und gibt die Helligkeit wieder.

Aufgrund ihrer hohen Festigkeit können die Zahnfrontverblendungskörper relativ dünn ausgeführt sein. Besonders bevorzugt ist dabei vorgesehen, dass der Zahnfrontverblendungskörper an einem seiner Endbereiche eine Schneidkante, vorzugsweise mit einer Dicke von 1,0mm bis 1,3mm, aufweist, wobei vorzugsweise vorgesehen ist, dass der Zahnfrontverblendungskörper außerhalb des Endbereichs mit der Schneidkante maximal eine Dicke von 0,6mm aufweist und/oder an seinem der Schneidkante gegenüberliegenden Ende flach ausläuft. Unter Dicke ist dabei jeweils die Wandstärke im entsprechenden Bereich des Zahnfrontverblendungskörpers zu verstehen.

Durch die erfindungsgemäßen Zahnfrontverblendungskörper wird es, wie gesagt, möglich, dass der Zahnarzt den Zahnfrontverblendungskörper bzw. einen entsprechenden Rohling vor Ort an die geometrischen Anforderungen des Zahns bzw. Zahnstumpfs des Patienten anpasst. Hat der Zahnarzt zusätzlich noch die Möglichkeit ein entsprechendes Befestigungsmaterial nach seiner Farbe auszusuchen, so kann er auch zusätzlich noch die gewünschte Farbanpassung vor Ort vornehmen. Um dem Zahnarzt, abgesehen von seiner normalen Ausstattung, alle für die Behandlung benötigten Hilfsmittel zur Verfügung zu stellen, sieht ein besonderer Aspekt der Erfindung ein Set mit mehreren, insbesondere verschieden großen und/oder verschieden geformten, erfindungsgemäßen Zahnfrontverblendungskörpern vor, wobei das Set zusätzlich zumindest ein, vorzugsweise flüssiges oder pastöses, Befestigungsmaterial und mehrere verschiedene Farben zum Einfärben des Befestigungsmaterials und/oder mehrere verschiedenfarbige, vorzugsweise flüssige oder pastöse, Befestigungsmaterialien aufweist, wobei das Befestigungsmaterial bzw. die Befestigungsmaterialien zum Befestigen, vorzugsweise Ankleben, des Zahnfrontverblendungskörpers an einem Zahn bzw. Zahnstumpf geeignet ist bzw. sind. Dies ermöglicht es, den Zahnarzt aus den ihm vom Set zur Verfügung gestellten Zahnfrontverblendungskörpern diejenige Größe auszusuchen, welche der natürlichen Situation am zu behandelnden Zahn oder Zahnstumpf am nächsten kommt. Die restliche Anpassung kann dann durch Bearbeiten bzw. Zuschleifen dieses Rohlings vor Ort vorgenommen werden. Hierzu kann der Zahnarzt auf die ihm standardmäßig zur Verfügung stehenden Schleifgeräte zurückgreifen. Weiters kann der Zahnarzt die geeignete Farbe bzw. das geeignet eingefärbte Befestigungsmaterial aussuchen, mit dem der Zahnfrontverblendungskörper am Zahn-stumpf bzw. Zahn befestigt, vorzugsweise angeklebt, wird, sodass auch die gewünschte Färbung des dann am Zahn bzw. Zahnstumpf befestigten Zahnfrontverblendungskörper erreicht ist. Um letztendlich verschiedenfarbiges Befestigungsmaterial zur Verfügung zu stellen, kann das Set in einer Ausgestaltungsform zumindest ein Befestigungsmaterial und mehrere verschiedene Farben aufweisen, mit denen das Befestigungsmaterial vom Zahnarzt eingefärbt wird. Bevorzugt ist jedoch vorgesehen, dass das Set bereits mehrere verschiedenfarbige Befestigungsmaterialien aufweist, bei welchen die Farbe nicht mehr vom Zahnarzt in das Befestigungsmaterial eingemischt werden muss. Das Befestigungsmaterial kann pastös, also breiig bzw. dickflüssig ausgebildet sein. Um kleinere Fissuren bearbeiten zu können, ist es aber auch denkbar, flüssiges Befestigungsmaterial im Set zur Verfügung zu stellen. Besonders bevorzugte Ausgestaltungsformen eines erfindungsgemäßen Sets sehen vor, dass das, vorzugsweise in pastöser Form vorliegende, Befestigungsmaterial dasselbe zusammengesetzte Material enthält wie die Zahnfrontverblendungskörper des Sets, allerdings eben noch in pastöser Konsistenz. Weiters ist es günstig, wenn das, insbesondere in flüssiger Form vorliegende, Befestigungsmaterial dasselbe organische Binde-mittel wie die Zahnfrontverblendungskörper des Sets, eben allerdings noch in flüssiger Form enthält.

Bevorzugte Ausgestaltungsformen des erfindungsgemäßen Sets sehen darüber hinaus vor, dass das Set zumindest einen Haftvermittler, vorzugsweise zumindest zwei verschieden Haftvermittler, und/oder zumindest ein Ätzmittel aufweist. Ein Haftvermittler bzw. das Bonding dienen dazu, die Verbindung zwischen dem hydrophilen Zahn und dem hydrophoben zusammengesetzen Material des Zahnfrontverblendungskörpers bzw. des Befestigungsmaterials herzustellen. Dabei sollte der Haftvermittler auch die Schrumpfkräfte des Befestigungsmaterials auffangen. Günstig ist es im Set zumindest zwei verschiedene Haftvermittler zur Verfügung zu stellen. Einer davon kann dann als Haftvermittler zwischen Zahn bzw. Zahnstumpf und Befestigungsmaterial und der andere Haftvermittler zwischen Befestigungsmaterial und Zahnfrontverblendungskörper eingesetzt werden. Als Haftvermittler sind z.B. die mit REF 2050 und REF 2051 bezeichneten Produkte der Firma Indigo-dental GmbH & Co. KG, in Pinneberg, Deutschland geeignet. Das Produkt REF 2050 kann z.B. als Haftvermittler zwischen Zahnstumpf und Befestigungsmaterial verwendet werden. Es enthält Methacrylierte Polyacrylsäure in einer Bis- GMA basierten Matrix. Das Produkt REF 2051 kann z.B. als Haftvermittler zwischen Befestigungsmaterial und Zahnfrontverblendungskörper verwendet werden. Es handelt sich dabei auch um ein Bis-GMA basiertes Methacrylat.

Das Ätzmittel z.B. in Form eines Ätzgels dient dazu, die durch den Bohrer des Zahnarztes verursachte Schmierschicht wegzuätzen und die Tubuli freizulegen. Geeignete Ätzmittel sind z.B. 15 -37%ige Phosphorsäure oder das Produkt REF 2052 der oben bereits genannten Firma. Es ist auch denkbar, bereits fertige Mischungen aus Ätzmittel und Haftvermittler einzusetzen. Generell kann es sich bei den Haftvermittlern um niedermolekulare Methacrylate handeln. Es können auch Monomere eingesetzt werden, welche ein hydrophiles und ein hydrophobes Ende haben, wie z.B. eine methacrylierte Polyacrylsäure.

Als zusammengesetztes Material für die Herstellung des Zahnfrontverblendungskörpers aber auch als Befestigungsmaterial kann z.B. das unter der Bezeichnung REF 2061 erhältliche Produkt der Firma Indigodental GmbH & Co. KG, in Pinneberg, Deutschland verwendet werden. Dieses Produkt enthält Triethylenglykoldimethacrylat, Urethandimethacrylat, Bis-GMA und ethoxyliertes Bisphenol A Dimethacrylat. Als organisches Bindemittel kann z.B. das bereits bezüglich der Haftvermittler genannte Produkt REF 2050 dieser Firma verwendet werden. Als Farbstoffe, insbesondere zum Einfärben des Befestigungsmaterials können z.B. organische Pigmente und/oder der anorganische Weißmacher TiO2 verwendet werden.

Weitere Einzelheiten und Merkmale bevorzugter Ausgestaltungsformen der Erfindung ergeben sich aus der nachfolgenden Figurenbeschreibung. Dabei zeigen:
Fig. 1 bis 3 verschiedene erfindungsgemäße Zahnfrontverblendungskörper für Zähne des Oberkiefers in Frontalansicht;
Fig. 4 bis 6 Längsschnitte durch den jeweils darüber angeordneten Zahnfrontverblendungskörper gemäß der Fig. 1 bis 3;
Fig. 7 bis 9 verschiedene erfindungsgemäße Zahnfrontverblendungskörper für Zähne des Unterkiefers;
Fig. 10 bis 12 Längsschnitte durch den jeweils darüber angeordneten Zahnfrontverblendungskörper gemäß der Fig. 7 bis 9;
Fig. 13 eine schematisierte Explosionsdarstellung zum Anbringen des Zahnfrontverblendungskörpers an einem Zahnstumpf;
Fig. 14 eine schematisierte Seitenansicht des am Zahnstumpf angebrachten Zahnfrontverblendungskörpers;
Fig. 15 eine schematisierte Mikroaufnahme des zusammengesetzten Materials des Zahnfrontverblendungskörpers und
Fig. 16 eine erfindungsgemäße Ausführungsform eines Sets.

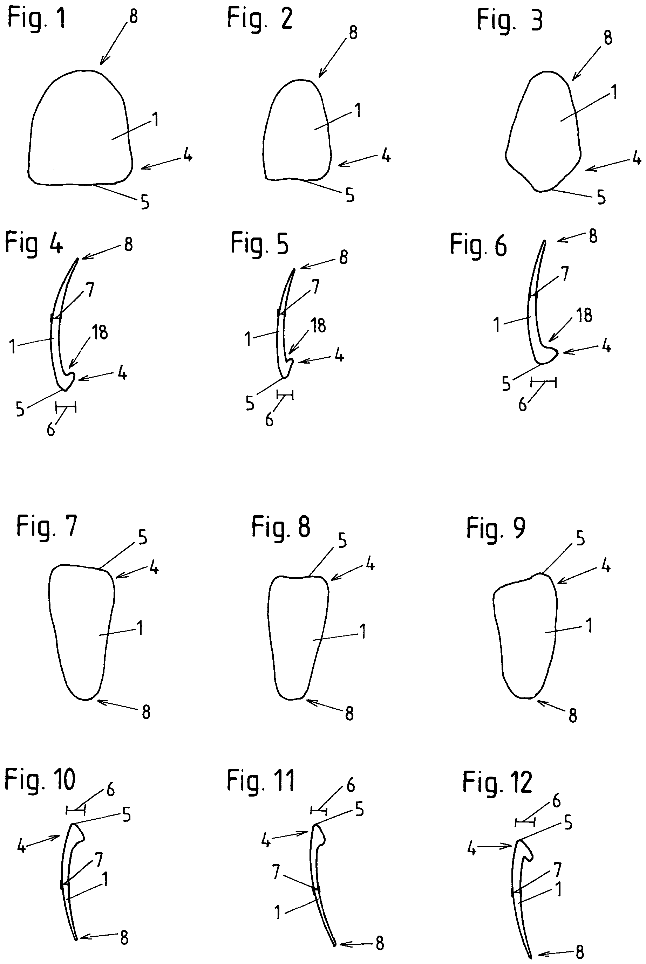

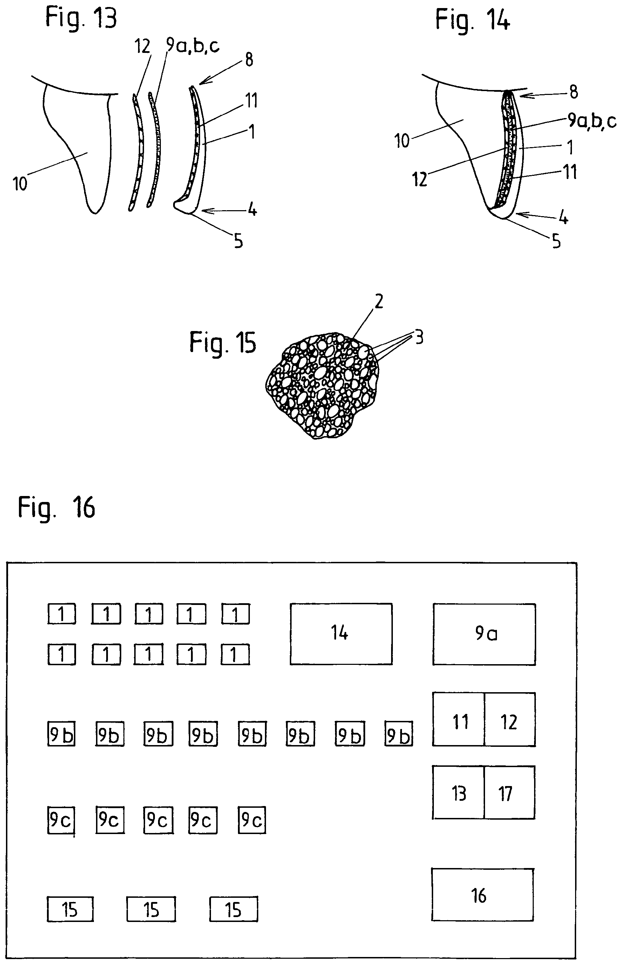

Die Fig. 1 bis 3 zeigen verschiedene Zahnfrontverblendungskörper 1 für verschiedene Zähne des Oberkiefers. Die Fig. 7 bis 9 zeigen verschiedene Zahnfrontverblendungskörper 1 für verschiedene Zähne des Unterkiefers. Untersuchungen haben gezeigt, dass es möglich ist, die bei unterschiedlichsten Patienten vorkommenden Zahnformen so zu generalisieren, dass man mit relativ wenigen verschieden vorgeformten Rohlingen der Zahnfrontverblendungskörper 1 fast alle natürlich vorkommenden Zahnformen abdecken kann, wenn der Zahnarzt vor Ort den richtigen Rohling des Zahnfrontverblendungskörpers 1 auswählt und an die tatsächlich gegebene Zahnform des Patienten anpasst, was bei erfindungsgemäßen Zahnfrontverblendungskörpern 1 möglich ist. Man kommt daher mit einer relativ geringen Anzahl an Sätzen von Rohlingen aus. Diese können z.B. in drei unterschiedlichen Größenstufen für den Oberkiefer und zwei unterschiedlichen Größenstufen für den Unterkiefer angeboten werden. Die Fig. 4 bis 6 und 10 bis 12 zeigen jeweils einen Längsschnitt durch den Zahnfrontverblendungskörper 1 der darüber angeordneten Figur. Jeder der dargestellten Zahnfrontverblendungskörper 1 weist einen Endbereich 4 im Bereich der Schneidkante 5 auf. Die Schneidkante 5 tritt beim Kauen jeweils direkt mit dem zu zerkauenden Nahrungsmittel in Kontakt um es zu zerteilen. Wie insbesondere in den Längsschnitten gemäß der Fig. 4 bis 6 und 10 bis 12 zu sehen ist, ist bei dem gezeigten Ausführungsbeispiel vorgesehen, dass der Zahnfrontverblendungskörper 1 im Endbereich 4 der Schneidkante 5 seine größte Dicke 6 aufweist. Die Dicke 6 liegt günstigerweise zwischen 1 mm und 1,3mm. Im Endbereich 4 sind die dargestellten Zahnfrontverblendungskörper 1 im Längsschnitt gesehen zumindest auf der Rückseite haken- bzw. stufenförmig ausgebildet, sodass die Schneidkante 5, wenn der Zahnfrontverblendungskörper 1 am Zahnstumpf 10 angebracht ist, die gesamte Schneidkante des so wieder hergestellten Zahnes bildet. Der Zahnstumpf 10 liegt, wie in Fig. 14 zu sehen, dann formschlüssig am freien Ende 18 des im Längsschnitt gesehen hakenförmig bzw. stufenförmig ausgebildeten Endbereichs 4 an. Außerhalb des Endbereichs 4 weist der Zahnfrontverblendungskörper 1 eine wesentlich geringere Dicke 7 auf. Diese erreicht günstigerweise maximal 0,6mm. Am der Schneidkante 5 gegenüberliegenden Ende 8 laufen die gezeigten Ausführungsbeispiele von Zahnfrontverblendungskörpern 1 flach aus. Insgesamt ergibt sich eine zumindest bereichsweise schalenförmig gewölbte Ausformung der Zahnfrontverblendungskörper 1.

Fig. 13 zeigt grob schematisiert einen bereits fertig präparierten und angeätzten Zahn-stumpf 10. Auf diesen wird zunächst ein erster Haftvermittler 12 aufgebracht, welcher einer innigen Verbindung zwischen Zahnstumpf 10 und Befestigungsmaterial 9a, b oder c dient. Günstigerweise handelt es sich bei dem Befestigungsmaterial um ein pastöses Material. Günstigerweise weist das Befestigungsmaterial dasselbe zusammengesetzte Material, also sowohl organisches Bindemittel 2 als auch Feststoffpartikel 3 auf, aus dem auch der Zahnfrontverblendungskörper 1 hergestellt wird, allerdings eben noch in pastöser Konsistenz. Zusätzlich ist das Befestigungsmaterial allerdings noch eingefärbt, um im Zusammenwirken mit der Transluzenz des Zahnfrontverblendungskörpers im fertig am Zahn befestigten Zustand die gewünschte Färbung zu ergeben. Für die großflächige Befestigung ist vor-zugsweise vorgesehen, dass das Befestigungsmaterial 9b und 9c sowohl organisches Bindemittel 2 als auch Feststoffpartikel 3 aufweist. Dieses Material ist allerdings relativ breiig bzw. pastös. Um Bereiche mit sehr feingliedriger Oberflächenstruktur bearbeiten zu können, kann anstelle dieses Befestigungsmaterials 9b und 9c auch ein Befestigungsmaterial 9a verwendet werden, welches abgesehen von der Einfärbung ausschließlich organisches Bindemittel 2 oder zumindest einen geringeren Anteil an Feststoffpartikeln 3, von z.B. 60 bis 70 Vol-% als Füllstoff aufweist. Dieses Befestigungsmaterial 9a kann dasselbe organische Bindemittel, vorzugsweise auf Methacrylatbasis, und auch dieselbe Art und Mischung von Feststoffpartikeln, aber eben in geringerer Konzentration, aufweisen, welche auch das zusammengesetzte Material des fertigen Zahnfrontverblendungskörpers 1 aufweist. Zwischen dem Befestigungsmaterial 9a, b oder c und dem Zahnfrontverblendungskörper 1 wird günstigerweise wiederum ein Haftvermittler 11 angebracht. Besonders bevorzugt handelt es sich dabei um einen anderen Haftvermittler 11 als den Haftvermittler 12. Ge-eignete Beispiele von Haftvermittlern wurden eingangs genannt.

Fig. 14 zeigt den fertigen Zustand, in dem der Zahnfrontverblendungskörper 1 mittels des Befestigungsmaterials 9a, b oder c am Zahnstumpf 10 unter Zwischenschaltung der Haftvermittler 11 und 12 befestigt ist. Die im Befestigungsmaterial 9a, b oder c vorhandenen Farbpigmente scheinen durch das transluzente Material des Zahnfrontverblendungskörpers 1 hindurch und geben so, ähnlich wie beim natürlichen Zahn, die gewünschte Färbung.

Fig. 15 ist eine schematisierte Vergrößerung des zusammengesetzten Materials des Zahnfrontverblendungskörpers 1. Die verschiedenen Feststoffpartikel 3 sind mittels des organischen Bindemittels 2 innig miteinander verbunden. Als organisches Bindemittel kommen vorzugsweise methacrylatbasierte Harze zum Einsatz. Ein konkretes Beispiel eines geeigneten organischen Bindemittels 2 ist weiter oben genannt. Die Feststoffpartikel 3 bestehen bevorzugt aus einer Glasmischung mit Feststoffpartikeln 3 unterschiedlicher Korngröße. Hierdurch wird eine sehr dichte Packung und damit eine hohe Stabilität und Abrasionsfestigkeit des zusammengesetzten Materials erreicht. Günstigerweise beträgt der Volumenanteil der Feststoffpartikel 3 im Zahnfrontverblendungskörper 1 zumindest 75%, vor-zugsweise zumindest 82%, am zusammengesetzten Material. Um auch die Lücken zwischen den relativ großen Körnern möglichst vollständig ausfüllen zu können, sehen besonders bevorzugte Ausgestaltungsformen der Erfindung vor, dass die Korngrößen zumindest eines Teils der Feststoffpartikel 3 zwischen 0,01 µm und 3 µm liegen.

Fig. 16 zeigt schematisiert ein Set mit mehreren erfindungsgemäßen Zahnfrontverblendungskörpern 1 in verschiedenen Größen und diversen anderen Hilfsmitteln, um dem Zahnarzt die Anpassung und das Anbringen des Zahnfrontverblendungskörpers 1 am Zahnstumpf 10 innerhalb eines einzigen Behandlungstermins zu ermöglichen. Das Set gemäß Fig. 16 weist zunächst verschiedene Sätze von verschieden großen und verschieden ausgeformten Zahnfrontverblendungskörpern 1 auf. Im gezeigten Ausführungsbeispiel gibt es jeweils einen Satz von Zahnfrontverblendungskörpern 1 für die Zähne des Oberkiefers in drei verschiedenen Größen. Für den Unterkiefer sind im gezeigten Set Sätze in zwei unterschiedlichen Größen vorgesehen. Die Zahnfrontverblendungskörper 1 sind dabei in verschiedenen Grundformen aufgebaut. Der Größenunterschied zwischen den kleinen, mittleren und größeren Ausgestaltungsformen beträgt vorzugsweise jeweils 10%. Der Zahnarzt kann aus diesem Angebot von Zahnfrontverblendungskörpern 1 den für den jeweiligen Zahn des Patienten am besten passenden aussuchen und die Feinanpassung dann mittels entsprechendem Zuschleifen vor Ort durchführen. Um dem Zahnarzt das Auswählen des richtigen Zahnfrontverblendungskörpers 1 zu erleichtern, weist das Set gemäß Fig. 16 verschiedene Formschablonen 15 auf, mit denen in einfacher Art und Weise zunächst der optimal vorgeformte Zahnfrontverblendungskörper 1 ausgewählt werden kann. Neben den Zahnfrontverblendungskörpern 1 weist das Set gemäß Fig. 16 noch verschiedenste Befestigungsmaterialien 9a, b und c auf. Bei 9a handelt es sich um, in verschiedene Spritzen abgefülltes, verschieden eingefärbtes organisches Bindemittel 2, gegebenenfalls mit einem geringen Anteil an Feststoffpartikeln 3, vorzugsweise in der oben genannten Konzentration. Das Befestigungsmaterial 9a wird dann eingesetzt, wenn es darum geht, sehr kleine Hohlräume mit Befestigungsmaterial zu füllen. Das Befestigungs-material 9a kann abgesehen von den Farbpigmenten und gegebenenfalls weiteren Zusätzen im Wesentlichen dem organischen Bindemittel 2, welches das zusammengesetzte Material der Zahnfrontverblendungskörper 1 dieses Sets aufweist, gegebenenfalls zusätzlich mit dem genannten geringen Anteil an Feststoffpartikeln 3, entsprechen. Bei den Befestigungsmaterialien 9b und 9c handelt es sich um verschieden eingefärbte pastöse Befestigungsmaterialien. Diese weisen günstigerweise das zusammengesetzte Material der Zahnfrontverblendungskörper 1 in noch nicht ausgehärteter Konsistenz auf. Die Befestigungsmaterialien 9b und 9c umfassen somit sowohl das organische Bindemittel 2 als auch die Feststoffpartikel 3. Der Zahnarzt kann mit Hilfe des Farbschlüssels 14 das geeignet eingefärbte Befestigungsmaterial 9a, b oder c auswählen und in der in Fig. 13 angedeuteten Art und Weise zum Befestigen des Zahnfrontverblendungskörpers 1 am Zahnstumpf 10 verwenden. Im gezeigten Ausführungsbeispiel sind die verschiedenen Befestigungsmaterialien 9c für Fälle vorgesehen, in denen am Zahnstumpf 10 noch natürlicher Zahnschmelz vorhanden ist. Die hier gewählten Farben sind daher sehr hell. Die verschiedenen Befestigungsmaterialien 9b sind für den Fall vorgesehen, dass der Zahnschmelz vom Zahnstumpf 10 vollständig entfernt ist und mit kräftigeren Farben gearbeitet werden muss, damit das gewünschte Ergebnis erreicht wird. Das Set gemäß Fig. 16 umfasst im gezeigten Ausführungsbeispiel auch Applikationsnadeln 16, welche dazu dienen, das Befestigungsmaterial 9a, b und/oder c auf den Zahnstumpf 10 aufzubringen. 11 und 12 sind zwei verschiedene Haftvermittler die, wie in Fig. 13 schematisiert gezeigt, verwendet werden können. Weiters umfasst das Set gemäß Fig. 16 ein Ätzmittel 13, welches dazu dient, vor Aufbringung des jeweiligen Haftvermittlers 11 bzw. 12 die durch die vorherige Bearbeitung des Zahnstumpfes 10 verursachte Schmierschicht wegzuätzen und die Tubuli bzw. Zahnstümpfe bzw. Zähne freizulegen. Im gezeigten Ausführungsbeispiel ist zusätzlich auch noch ein Mittel 17 vorgesehen, welches eine Kombination aus einem Ätzmittel und einem Haftvermittler ist. Bevorzugte Ausgestaltungsformen von entsprechenden Sets werden in entsprechenden Behältern angeboten. Die Aufteilung der Fächer und der Inhalt sind in Fig. 16 natürlich nur beispielhaft und schematisiert dargestellt.

Ein Verfahren zur Herstellung erfindungsgemäßer Zahnfrontverblendungskörper 1 aus dem z.B. im Handel erhältlichen, zusammengesetzten und eingangs genannten Material kann folgende Schritte vorsehen:
Zunächst wird das zusammengesetzten Material bzw. Composite, umfassend das organische Bindemittel 2 und die Feststoffpartikel 3, unter Vakuum in Kartuschen gefüllt, auf ca. 60° Celsius erwärmt und mit 80 bis 100bar Druck in die entsprechend einseitig transparent ausgeführten Zahnformen injiziert. Die Zahnformen können aus Glas und Chromstahl hergestellt sein. Die Aushärtung des zusammengesetzten Materials kann dann mit einem geeigneten LED-Licht und einer Belichtungszeit von z.B. 60 Sekunden erfolgen. Ein geeignetes LED-Licht ist z.B. charakterisiert durch eine Wellenlänge von 450 Nanometern (nm) bis 480 nm. Danach können die Zahnfrontverblendungskörper 1 in einem Vakuumdruckluftofen über einen Zeitraum von z.B. zehn Minuten auf z.B. 100° erwärmt und thermisch vergütet werden. Im Anschluss daran werden die Rohlinge der Zahnfrontverblendungskörper 1 aus den Zahnformen herausgenommen. Erfindungsgemäß ist vorgesehen, dass an der fazialen Fläche bzw. Oberfläche des Zahnfrontverblendungskörpers 1 eine Laserlichtbearbeitung stattfindet. Anschließend kann ein Zuschneiden oder Nachbearbeiten der Form erfolgen. Bei der Laserbearbeitung der fazialen Flächen wird das organische Bindemittel 2 an der Oberfläche des Zahnfrontverblendungskörpers 1 inhibiert bzw. entfernt und es entsteht eine so gut wie ausschließlich auf den Feststoffpartikeln 3 basierende hochglänzende Oberfläche, welche nach Abschluss der Laserbehandlung eine durchgehende ausgehärtete bzw. verglaste Schmelzschicht als Oberfläche des Zahnfrontverblendungskörpers 1 bildet. Diese ausgehärtete bzw. verglaste Schmelzschicht ist in einem Schnitt durch den Zahnfrontverblendungskörpers 1 nachweisbar und weist bevorzugt Dicken zwischen 10 und 20 Mikrometer (µm) auf.

Alternativ zum geschilderten Herstellungsverfahren ist es auch möglich, aus einem Block aus dem zusammengesetzten Material den Zahnfrontverblendungskörper 1 herauszulasern, wobei sich automatisch auch die ausgehärtete bzw. verglaste Schmelzschicht als Oberfläche des Zahnfrontverblendungskörpers 1 bildet.

### Legende zu den Hinweisziffern:

- 1: Zahnfrontverblendungskörper
- 2: Bindemittel
- 3: Feststoffpartikel
- 4: Endbereich
- 5: Schneidekante
- 6: Dicke
- 7: Dicke
- 8: Ende
- 9a, b, c: Befestigungsmaterial
- 10: Zahnstumpf
- 11: Haftvermittler
- 12: Haftvermittler
- 13: Ätzmittel
- 14: Farbschlüssel
- 15: Formschablonen
- 16: Applikationsnadel
- 17: Ätzmittel und Haftvermittler
- 18: freies Ende

## Patentansprüche

1. Zahnfrontverblendungskörper (1), bestehend aus einem zusammengesetzten Material, wobei das zusammengesetzte Material zumindest ein organisches Bindemittel (2) und anorganische Feststoffpartikel (3) als Füllstoff enthält, **dadurch gekennzeichnet, dass** eine äußere Oberfläche des Zahnfrontverblendungskörpers (1) zumindest bereichsweise laserbehandelt ist und eine durchgehende ausgehärtete bzw. verglaste Schmelzschicht bildet, welche im Wesentlichen nur aus dem verschmolzenen Material der Feststoffpartikel (3) besteht.

2. Zahnfrontverblendungskörper (1) nach Anspruch 1, **dadurch gekennzeichnet, dass** er zumindest bereichsweise schalenförmig gewölbt ausgebildet ist.

3. Zahnfrontverblendungskörper (1) nach Anspruch 1 oder 2, **dadurch gekennzeichnet, dass** die Feststoffpartikel (3) des Füllstoffs Glas, vorzugsweise eine Mischung verschiedener Glasarten, aufweisen oder daraus bestehen, und/oder dass der Füllstoff Feststoffpartikel (3) mit voneinander verschiedenen Korngrößen, insbesondere mit Korngrößen zwischen 0,01µm und 3µm, aufweist.

4. Zahnfrontverblendungskörper (1) nach einem der Ansprüche 1 bis 3, **dadurch gekennzeichnet, dass** die Feststoffpartikel (3) des Füllstoffs einen Anteil von zumindest 75 Vol-%, vorzugsweise von zumindest 82 Vol-%, am zusammengesetzten Material des Zahnfrontverblendungskörpers (1) haben.

5. Zahnfrontverblendungskörper (1) nach einem der Ansprüche 1 bis 4, **dadurch gekennzeichnet, dass** er transluzent ist, vorzugsweise eine Transluzenz von 28% bis 39% aufweist, und/oder einen L-Wert von 59 bis 69 aufweist und/oder einen a-Wert von -0,35 bis -3,2 aufweist und/oder einen b-Wert von -0,4 bis -6,95 aufweist.

6. Zahnfrontverblendungskörper (1) nach einem der Ansprüche 1 bis 5, **dadurch gekennzeichnet, dass** er an einem seiner Endbereiche (4) eine Schneidkante (5), vorzugsweise mit einer Dicke (6) von 1mm bis 1,3 mm, aufweist, wobei vorzugsweise vorgesehen ist, dass der Zahnfrontverblendungskörper (1) außerhalb des Endbereichs (4) mit der Schneidkante (5) maximal eine Dicke (7) von 0,6mm aufweist und/oder an seinem der Schneidkante gegenüberliegenden Ende (8) flach ausläuft.

7. Zahnfrontverblendungskörper (1) nach einem der Ansprüche 1 bis 6, **dadurch gekennzeichnet, dass** das organische Bindemittel (2) Methacrylat aufweist.

8. Set mit mehreren, insbesondere verschieden großen und/oder verschieden geformten, Zahnfrontverblendungskörpern (1) nach einem der Ansprüche 1 bis 7, wobei das Set zusätzlich zumindest ein, vorzugsweise flüssiges oder pastöses, Befestigungsmaterial (9a, 9b, 9c) und mehrere verschiedene Farben zum Einfärben des Befestigungsmaterials (9a, 9b, 9c) und/oder mehrere verschiedenfarbige, vorzugsweise flüssige oder pastöse, Befestigungsmaterialien (9a, 9b, 9c) aufweist, wobei das Befestigungsmaterial (9a, 9b, 9c) bzw. die Befestigungsmaterialien (9a, 9b, 9c) zum Befestigen, vorzugsweise Ankleben, des Zahnfrontverblendungskörpers (1) an einem Zahnstumpf geeignet (10) ist bzw. sind.

9. Set nach Anspruch 8, **dadurch gekennzeichnet, dass** das Befestigungsmaterial (9) dasselbe zusammengesetzte Material oder dasselbe organische Bindemittel enthält wie die Zahnfrontverblendungskörper (1).

10. Set nach Anspruch 8 der 9, **dadurch gekennzeichnet, dass** das Set zumindest einen Haftvermittler (11,12), vorzugsweise zumindest zwei verschiedene Haftvermittler (11,12), und/oder zumindest ein Ätzmittel (13) aufweist.

## Claims

1. Tooth front veneer (1) consisting of a composite material, said composite material containing at least one organic binder (2) and inorganic solid particles (3) as filler, **characterised in that** an outer surface of the tooth front veneer (1) is, at least in certain regions, laser-treated and forms a continuous hardened or glazed enamel layer which substantially consists only of the melted material of the solid particles (3).

2. Tooth front veneer (1) according to claim 1, **characterised in that**, at least in certain regions, it has a convex, shell-like form.

3. Tooth front veneer (1) according to claim 1 or 2, **characterised in that** the solid particles (3) of the filler contain or consist of glass, preferably a mixture of different types of glass, and/or that the filler contains solid particles (3) with different particle sizes, in particular with particle sizes between 0.01 µm and 3 µm.

4. Tooth front veneer (1) according to one of the claims 1 to 3, **characterised in that** the solid particles (3) of the filler make up at least 75 vol%, preferably at least 82 vol%, of the composite material of the tooth front veneer (1).

5. Tooth front veneer (1) according to one of the claims 1 to 4, **characterised in that** it is translucent, preferably having a translucency of 28% to 39%, and/or an L value of 59 to 69 and/or an a value of -0.35 to -3.2 and/or a b value of -0.4 to -6.95.

6. Tooth front veneer (1) according to one of the claims 1 to 5, **characterised in that** it has on one of its end regions (4) an incisal edge (5), preferably with a thickness (6) of 1 mm to 1.3 mm, wherein the tooth front veneer (1) preferably has a maximum thickness (7) of 0.6 mm outside of the end region (4) with the incisal edge (5) and/or tapers off towards its end opposite the incisal edge (8).

7. Tooth front veneer (1) according to one of the claims 1 to 6, **characterised in that** the organic binder (2) comprises methacrylate.

8. Set comprising several, in particular different-sized and/or different-shaped, tooth front veneers (1) according to one of the claims 1 to 7, wherein the set includes in addition at least one, preferably liquid or paste-like, bonding material (9a, 9b, 9c) and several different colourings to colour the bonding materials (9a, 9b, 9c) and/or several different-coloured, preferably liquid or paste-like, bonding materials (9a, 9b, 9c), wherein the bonding material (9a, 9b, 9c) or bonding materials (9a, 9b, 9c) is or are suitable for fixing, preferably adhesively bonding, the tooth front veneer (1) onto a tooth stump (10).

9. Set according to claim 8, **characterised in that** the bonding material (9) contains the same composite material or the same organic binder as the tooth front veneer (1).

10. Set according to claim 8 or 9, **characterised in that** the set includes at least one bonding agent (11, 12), preferably at least two different bonding agents (11, 12), and/or at least one caustic agent(13).

## Revendications

1. Corps de facette dentaire (1), constitué d'un matériau composite, ledit matériau composite contenant au moins un liant organique (2) et des particules solides minérales (3) en tant que charge, **caractérisé en ce qu'**une surface extérieure du corps de facette dentaire (1) est traitée par laser au moins par endroit et forme une couche d'émail durcie ou vitrifiée continue, laquelle est constituée essentiellement que du matériau fondu des particules solides (3).

2. Corps de facette dentaire (1) selon la revendication 1, **caractérisé en ce qu'**il est au moins par endroit incurvé en forme de coquille.

3. Corps de facette dentaire (1) selon la revendication 1 ou 2, **caractérisé en ce que** les particules solides (3) de la charge comprennent ou sont constituées de verre, de préférence d'un mélange de différents types de verres, et/ou **en ce que** la charge présente des particules solides (3) de différentes granulométries, en particulier de granulométries comprises entre 0,01 µm et 3 µm.

4. Corps de facette dentaire (1) selon l'une des revendications 1 à 3, **caractérisé en ce que** les particules solides (3) de la charge représentent au moins 75 % en volume, de préférence au moins 82 % en volume, du matériau composite du corps de facette dentaire (1).

5. Corps de facette dentaire (1) selon l'une des revendications 1 à 4, **caractérisé en ce qu'**il est translucide, de préférence présentant une translucidité comprise entre 28 % et 39 %, et/ou présentant une valeur L comprise entre 59 et 69, et/ou présentant une valeur a comprise entre -0,35 et -3,2, et/ou présentant une valeur b comprise entre -0,4 et -6,95.

6. Corps de facette dentaire (1) selon l'une des revendications 1 à 5, **caractérisé en ce qu'**il présente à l'une de ses zones d'extrémité (4) une arête coupante (5), de préférence d'une épaisseur (6) comprise entre 1 mm et 1,3 mm, le corps de facette dentaire (1) présentant de préférence une épaisseur maximale (7) de 0,6 mm en dehors de la zone d'extrémité (4) avec l'arête coupante (5), et/ou se termine à son extrémité (8) opposée à l'arête coupante par un tronçon plat.

7. Corps de facette dentaire (1) selon l'une des revendications 1 à 6, **caractérisé en ce que** le liant organique (2) contient du méthacrylate.

8. Kit comprenant plusieurs corps de facette dentaire (1), notamment de tailles et/ou de formes différentes, selon l'une des revendications 1 à 7, le kit comprenant en outre au moins un matériau de fixation (9a, 9b, 9c), de préférence liquide ou pâteux, et plusieurs couleurs différentes pour colorer le matériau de fixation (9a, 9b, 9c) et/ou plusieurs matériaux de fixation (9a, 9b, 9c) de couleurs différentes, de préférence liquides ou pâteux, le ou les matériaux de fixation (9a, 9b, 9c) étant apte(s) à fixer, de préférence à coller, le corps de facette dentaire (1) sur un moignon dentaire (10).

9. Kit selon la revendication 8, **caractérisé en ce que** le matériau de fixation (9) contient le même matériau composite ou le même liant organique que le corps de facette dentaire (1).

10. Kit selon la revendication 8 ou 9, **caractérisé en ce que** le kit comprend au moins un promoteur d'adhérence (11, 12), de préférence au moins deux promoteurs d'adhérence (11, 12) différents, et/ou au moins un produit décapant (13).
